Europäisches Patentamt

European Patent Office

Office européen des brevets

(11) Publication number: **0 259 006 B1**

## EUROPEAN PATENT SPECIFICATION

(45) Date of publication of patent specification: **12.05.93**　(51) Int. Cl.⁵: **A61B 5/04**, A61N 1/04

(21) Application number: **87306810.0**

(22) Date of filing: **31.07.87**

(54) **Flat, conformable, biomedical electrode.**

(30) Priority: **01.08.86 US 892691**
**03.10.86 US 915138**
**01.08.86 US 892506**

(43) Date of publication of application:
**09.03.88 Bulletin 88/10**

(45) Publication of the grant of the patent:
**12.05.93 Bulletin 93/19**

(84) Designated Contracting States:
**DE FR GB IT SE**

(56) References cited:
**EP−A− 0 097 436**
**FR−A− 1 470 698**
**US−A− 4 524 775**

(73) Proprietor: **MINNESOTA MINING AND MANU−
FACTURING COMPANY**
**3M Center, P.O. Box 33427**
**St. Paul, Minnesota 55133−3427(US)**

(72) Inventor: **Roberts, Charles William Minnesota
Mining and**
**Manufacturing Co 2501 Hudson Road P.O.
Box 33427**
**St.Paul Minnesota 55133−3427(US)**
Inventor: **Strand, Jerome Elroy Minnesota
Mining and**
**Manufacturing Co 2501 Hudson Road P.O.
Box 33427**
**St.Paul Minnesota 55133−3427(US)**

(74) Representative: **Baillie, Iain Cameron et al
c/o Ladas & Parry, Altheimer Eck 2
W−8000 München 2 (DE)**

Rank Xerox (UK) Business Services
(3. 10/3.6/3.3. 1)

## Description

Technical Field

The present invention relates generally to biomedical electrodes.

Background Art

Biomedical electrodes are useful for both stimulation and body monitoring functions. Stimulation uses of biomedical electrodes include transcutaneous electronic nerve stimulation (TENS) for the treatment of pain and neuromuscular stimulation (NMS) as, for example, treatment for scoliosis. Body monitoring uses for biomedical electrodes include electrocardiogram (ECG) for monitoring hear activity.

Among biomedical electrodes in existence are those of Phipps et al., Cartmell and Larimore. Phipps et al in United States Patent No. 3,170,459 discloses a biomedical instrumentation electrode constructed from multiple plies of discs made from a relatively inflexible material, i.e., cork. The electrode utilizes a conductive gel to establish electrical contact with the body. Cartmell in United States Patent No. 4,543,958 discloses a medical electrode assembly. The electrode has a flexible, dimensionally stable substrate which is striped with an electrically conductive paint. The electrode is then clamped into a bulky cable connector. Larimore in United States Patent No. 4,458,696 (assigned to Minnesota Mining and Manufacturing Company) discloses a TENS electrode with a raised structure to permit entry of and attachment to a tubular electrical conductor.

These electrodes suffer from several deficiencies including that all are "high profile" electrodes and that the electrodes do not "conform" well to the body.

EP−A−0097436 to Minnesota Mining and Manufacturing Company discloses a biomedical electrode useful for delivering current to the body. The electrode has a non−metallic electrically conductive film located between a thin, conformable, electrically conductive metal layer and the skin. The electrode also has an extensible backing overlying the metal layer and an electrically conductive pressure sensitive adhesive.

Disclosure of Invention

The present invention provides a biomedical electrode which has flexibility in the lead wire insertion direction.

According to a first aspect of the invention there is provided a biomedical electrode adapted to be applied to a body and adapted to be electrically and mechanically connected to an electrical lead wire comprising:

a protective, electrically insulative web;

a first layer of adhesive which is electrically conductive positioned adjacent one side of said protective, insulative web, said one side adapted to be oriented toward said body; and

a removable release liner between said protective, electrically insulative web and said first layer of adhesive, said release liner having a release agent facing said first layer of adhesive, said release liner covering only a portion of the surface area of said protective, electrically insulative web.

According to a second aspect of the invention there is provided a biomedical electrode adapted to be applied to a body, comprising:

a backing material having an aperture, having a top side adapted to be oriented away from said body and having a bottom side adapted to be oriented toward said body;

a protective web at least partially secured to said top side of said backing material and covering said aperture;

a first removable liner positioned between said protective web and said backing material, said first removable liner covering at least a portion of the area of said backing material covered by said protective web and covering said aperture;

a first layer of adhesive which is electrically conductive positioned adjacent said bottom side of said backing material and adapted to be applied to said body;

a second removable liner positioned adjacent said first layer of adhesive opposite from said backing material;

a lead wire having one end adapted to be positioned within said aperture from said top side of said backing material so as to be adhered to said first layer of adhesive; and

whereby said second removable liner may be removed and said biomedical electrode may be applied

2

EP 0 259 006 B1

to said body with said first layer of adhesive and whereby said first removable liner may be removed and said lead wire may be positioned with said one end within said aperture and secured by said protective web.

Brief Description of Drawings

The advantages, construction and operation of the present invention will become more readily apparent from the following description and accompanying drawings in which:

Figure 1 is an isometric view of the biomedical electrode of the present invention.

Figure 2 is a side view of an embodiment of the biomedical electrode of the present invention with a double coated release liner and electrical lead wire illustrated;

Figure 3 is a side view of an embodiment of the biomedical electrode of the present invention with an extended release liner;

Figure 4 is a side view of an embodiment of the biomedical electrode of the present invention with a center located release liner;

Figure 5 is a side view of an embodiment of the biomedical electrode of the present invention with a single sided folded release liner;

Figure 6 is a side view of an embodiment of the biomedical electrode of the present invention with an extended release liner and a protective web;

Figure 7 is a side view of an embodiment of the biomedical electrode of the present invention;

Figure 8 is a side view of an alternative embodiment of the biomedical electrode of the present invention;

Figure 9 is an isometric view of the biomedical electrode with the removable release liner being removed and the electrical lead wire being inserted.

Figure 10 is an expanded side view of the biomedical electrode; and

Figure 11 is an isometric view of the biomedical electrode attached to the body.

Detailed Description

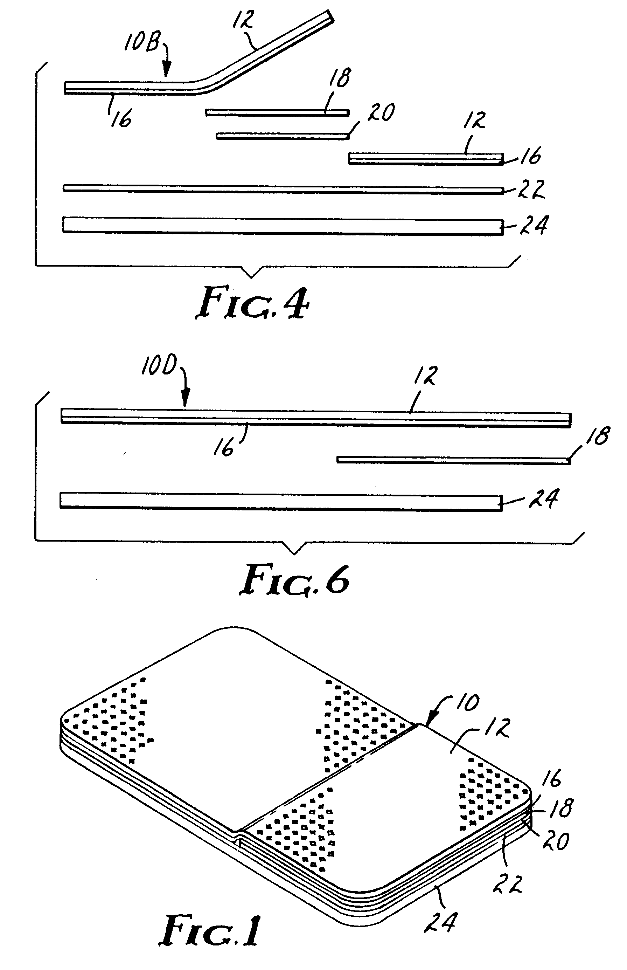

Figure 1 illustrates an isometric view of a preferred embodiment view of the biomedical electrode 10 of the present invention. The biomedical electrode 10 is also shown in expanded side view of Figure 2. The top of the biomedical electrode 10 is a protective, electrically insulative web 12. Web 12 protects the top of biomedical electrode 10 from physical damage and covers lead wire 14 when it is inserted in the biomedical electrode 10 to help secure lead wire 14 in place. Web 12 is electrically insulative to confine the electrical signals used in the biomedical electrode to the lead wire 14 or to the body (not shown). As shown in Figure 1, web 12 is preferably perforated making web 12 more conformable to the body contour and for esthetics. The perforations also assist in the tearability of web 12 once it is desired to remove lead wire 14 from the biomedical electrode 10. In a preferred embodiment, web 12 is approximately 4 mils (0.1 millimeters) thick and is constructed from a pigmented low molecular weight polyethylene film.

Web 12 is attached to the remainder of biomedical electrode 10 with a pressure sensitive adhesive 16. In a preferred embodiment, the pressure sensitive adhesive 16 is an acrylate adhesive. A release liner 18, which is removable, is placed beneath pressure sensitive adhesive 16 covering a portion of the surface area of biomedical electrode 10. Release liner 18 facilitates the lifting of web 12 away from the remainder of the biomedical electrode 10 in order that lead wire 14 may be inserted and web 12 subsequently reapplied securing lead wire 14 in biomedical electrode 10. In a preferred embodiment, release liner 18 is a Polyslick™ material as manufactured by James River Corporation, H.P. Smith Division, Bedford Park, Illinois. A conductive adhesive 20 is positioned in the biomedical electrode 10 below release liner 18. Conductive adhesive 20 need only cover a portion of the surface area of biomedical electrode 10 to which lead wire 14 may be positioned. In a preferred embodiment, conductive adhesive 20 covers approximately the same surface area of biomedical electrode 10 as does release liner 18. This allows for relative general ease in the positioning of lead wire 14 once release liner 18 has been removed. In a preferred embodiment, conductive adhesive 20 is made conductive through the inclusion of silver particles. In a preferred embodiment, a resistivity measurement using a one inch square brass plate above and below the adhesive measures approximately 0.01 ohms. The exact conductivity of conductive adhesive 20, of course, depends upon the end use to which biomedical electrode 10 is intended. Generally, it is expected that resistivities of conductive adhesive may generally range up to one ohm. In another embodiment, approximately ten ohms may be sufficient and higher resistivities may be allowable in other embodiments and for other uses for biomedical electrode 10. Conductive adhesive, especially conductive adhesives containing silver particles are widely available. An example of a suitable adhesive is a solvent based acrylate adhesive containing

3

silver particles about 3 mils (0.076 mm) in diameter which is coated through a knife coater at about 12 mils (0.305 mm) thickness. The coated adhesive is heated to drive off the solvent resulting in an adhesive layer of about 1.0 mils (0.025 mm) in thickness. Note that the silver particles are larger than the thickness of the resulting adhesive giving the layer its needed electrical conductivity. An adhesive similar to this but which has been coated on aluminum is available as X1170 foil tape from Minnesota Mining and Manufacturing Company, St. Paul, Minnesota. Below conductive adhesive 20 an electrically conductive film 22 , preferably a metal vapor coated conductive film, covers a large portion of the surface area of biomedical electrode and, in a preferred embodiment, covers the entire surface of biomedical electrode 10. The purpose of electrically conductive film 22 is to disburse the current delivered by or received by biomedical electrode 10 over a larger portion of the surface area of biomedical electrode 10. Electrically conductive film 22 may also operate to provide an ion barrier between the insertable lead wire 14 and the body. In a preferred embodiment, the electrically conductive film is an ethylene vinyl acetate loaded with approximately 28% carbon or a, Velostat® film manufactured by Minnesota Mining and Manufacturing Company, Saint Paul, Minnesota in either case coated top side with a $7 \times 10^{-8}$ to $1.2 \times 10^{-7}$ meters thick vapor coat of aluminum or silver. In a preferred embodiment, electrically conductive film 22 is approximately 3 mils (0.076 millimeters) thick. As indicated, conductive adhesive 24 operates to secure biomedical electrode 10 to the body to biomedical electrode 10 or vice versa. It is preferred that conductive adhesive 24 have better cohesion than adhesion in order to facilitate the ease in which the biomedical electrode 10 may be removed from the body. Conductive adhesive 24 may generally have a volume resistivity of the range from 50 to 200 ohm – centimeters although lower and high volume resistivities may work for some uses of the biomedical electrode 10. In a preferred embodiment, conductive adhesive 24 is from 10 mils (0.25 millimeters) to 60 mils (1.52 millimeters) thick. In a reuseable electrode it is preferred that the conductive adhesive 24 be from 30 mils (0.76 mm) to 44 mils (1.12 mm) thick. In a disposable electrode it is preferred that the conductive adhesive be from 10 mils (0.25 mm) to 24 mils (0.63 mm) thick. An example of a conductive adhesive 24 desirable for a reuseable electrode is described in US – A – 3865770. Blake, Water – Dispersable Pressure – Sensitive Adhesive, Tape Made Therewith, and Novel Tackifiers Therefore. It is preferred that the adhesive in Blake be modified and the following ingredients used:

| Ingredient | Dry Weight Grams | Dry Weight Percent |
|---|---|---|
| Copolymer: Butyl Acrylate & Acrylic Acid in 3:1 ratio | 83.177 | 40.945 |
| Glycerin | 20 | 9.8452 |
| Butanediol | 20 | 9.8452 |
| Sorbitol | 20 | 9.8452 |
| Methyl diethanolamine (MDEA) | 28 | 13.783 |
| Potassium Chloride (KCl) | 6.9678 | 3.4300 |
| Foral AX (hydrogenated wood rosin) | 25 | 12.306 |
| Total | 203.14 | 100.04 |

An example of a conductive adhesive 24 desirable for a disposable electrode is described in U.S. Patent 4,554,924, Engel, Conductive Adhesive and Biomedical Electrode. It is preferred that the adhesive in Engel be modified and the following ingredients be used:

| Ingredient | Dry Weight Grams | Dry Weight Percent |
|------------|---------|---------|
| 1,4 Butanediol | 45 | 6.3993 |
| Glycerin | 75 | 10.665 |
| Sorbitol | 290 | 41.240 |
| K739, Polyacrylic | 17 | 2.4175 |
| Potassium Hydroxide | 3.25 | 0.46217 |
| Total Water | 155 | 22.042 |
| Acrylic Acid | 115 | 16.354 |
| Irgacure | 0.51635 | 0.07343 |
| Tegbm | 2.3186 | 0.32972 |
| Methyl ethyl hydroquinone | 0.12 | 0.01706 |
| Total | 703.20 | 100.04 |

Optionally, a second release liner (not shown) may be provided below conductive adhesive 24 to facilitate transportation and storage of biomedical electrode 10 before use or between uses.

Biomedical electrode 10 may be utilized by applying the biomedical electrode 10 to a body being secured by conductive adhesive 24. The protective, electrically insulative web 12 may be lifted since release liner 18 prevents the protective, electrically insulative web 12 from sticking to conductive adhesive 20. Once the protective insulative web 12 is lifted, release liner 18 may be removed and discarded. At this time, lead wire 14 may be inserted so that noninsulated portion 26 of lead wire 14 is positioned over conductive adhesive 20. Lead wire 14 also has an insulative portion 28 which extends from beneath the confines of protective, electrically insulative web 12 and may be connected to suitable electronic equipment intended to utilize biomedical electrode 10. Lead wire 14 may be a copper wire whose insulated portion 28 is insulated with any suitable insulation, as for example, rubber or plastic. The end of noninsulated portion 26 of lead wire 14 is a flat crimped on conductor plate 30. Conductor plate 30 is flat which facilitates the biomedical electrode 10 being of low profile, flat and conformable to the body. Conductor plate 30 may, for example, be a zinc plated copper or, optionally, a silver plated copper with a chloride treatment.

Figure 3 illustrates an alternative embodiment for the biomedical electrode 10A of the present invention. The construction of the biomedical electrode 10A in Figure 3 is similar to the biomedical electrode 10 in Figure 2 except that protective, electrically insulative web 12, pressure sensitive adhesive 16 and release liner 18 extend from one edge of conductive adhesive 20, electrically conductive film 22 and conductive adhesive 24. Having these items extend beyond the edge facilitates the lifting of protective, electrically insulative web 12 in order that release liner 18 may be removed and lead wire 14 inserted.

Figure 4 illustrates another expanded side view of another embodiment of the biomedical electrode 10B of the present invention. The embodiment illustrated in Figure 4 is similar with that as biomedical electrode 10 illustrated in Figure 2 with the exception that the portion of the surface area of biomedical electrode 10 which is covered by release liner 18 and conductive adhesive 20 is positioned centrally in the biomedical electrode 10. This embodiment illustrates that protective insulative web 12, as well as pressure sensitive adhesive 16 may be separated into two portions. Biomedical electrode 10B as illustrated in Figure 4 operates as before, protective insulative web 12 is lifted, release liner 18 is removed and lead wire 14 is inserted over conductive adhesive 20.

In the embodiments of biomedical electrode illustrated in Figures 1 – 4, a single sheet release liner 18 is illustrated. In these embodiments, release liner 18 is coated both sides with a release agent allowing the release liner 18 to release both from pressure sensitive adhesive 16 and conductive adhesive 20.

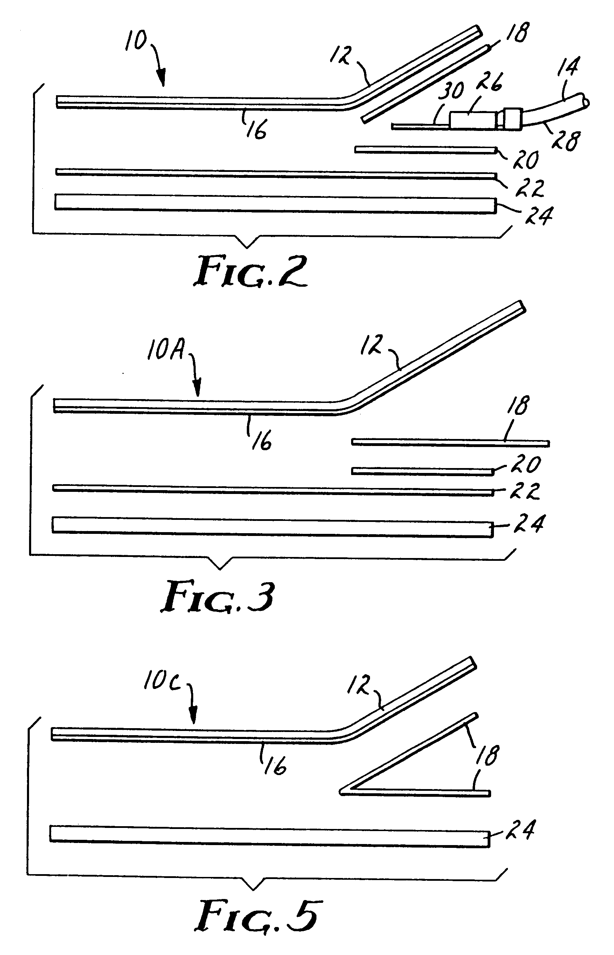

Another embodiment of biomedical electrode 10C is illustrated in a side exploded view in Figure 5. The biomedical electrode 10C illustrated in Figure 5 also contains a protective electrically insulative web 12 and a pressure sensitive adhesive 16. The biomedical electrode 10 also contains a release liner 18. Release liner 18 is illustrated as being folded with the closed side fold being positioned interiorly with respect to biomedical electrode 10 and the open side fold being positioned near an edge of the protective, electrically insulative web 12. The release liner 18 as illustrated in Figure 5 may be coated on only one side, the outside of release liner 18 as it is folded so that the release agent still contacts pressure sensitive adhesive 16 and conductive adhesive 24. As in the other embodiments, the biomedical electrode 10 C illustrated in Figure 5 contains conductive adhesive 24 preferably along the entire bottom surface area of biomedical electrode 10. Conductive adhesive 24 is the same as and performs the same purpose as conductive adhesive 24 in Figures 1 – 4. Note that the biomedical electrode 10C illustrated in Figure 5 is missing electrically conductive adhesive 20 and electrically conductive film 22. As noted earlier, electrically conductive film 22 was used to facilitate the dispersion of electrical currents over the entire surface of biomedical electrode. In situations where such dispersion is not required or such dispersion is not required to be achieved as well as can be achieved with the electrically conductive film 22, the electrically conductive film 22 may be omitted. With the omission of electrically conductive film 22, electrically conductive adhesive 20 is also no longer needed. Electrically conductive adhesive 20 was utilized to secure lead wire 14 when it was positioned within biomedical electrode 10. With the embodiment of the biomedical electrode 10C illustrated in Figure 5 when release liner 18 is removed, lead wire 14 may be inserted in its place and is secured between protective insulative web 12 by pressure sensitive adhesive 16 and conductive adhesive 24.

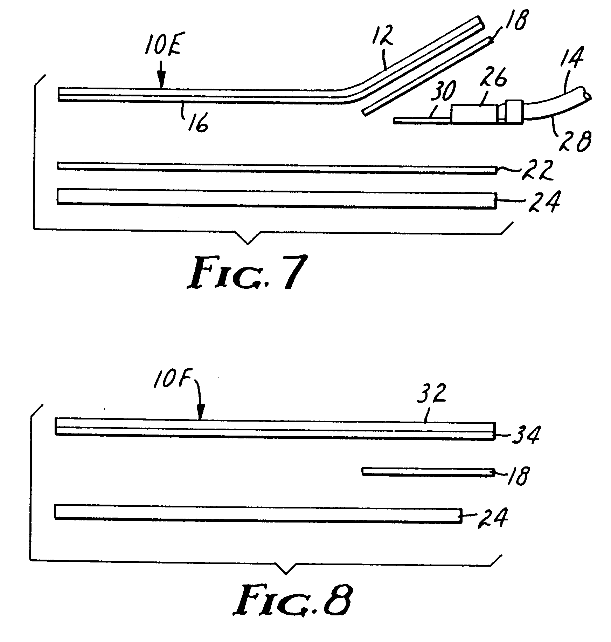

The biomedical electrode 10D as illustrated in Figure 6 is constructed similarly to the embodiment of biomedical electrode 10C illustrated in Figure 5 with the exception being that protective insulative web 12, pressure sensitive adhesive 16 and release liner 18 extend beyond an edge of conductive adhesive 24 (as in Figure 3) to facilitate lifting of protective insulative web 12 and removal of release liner 18.

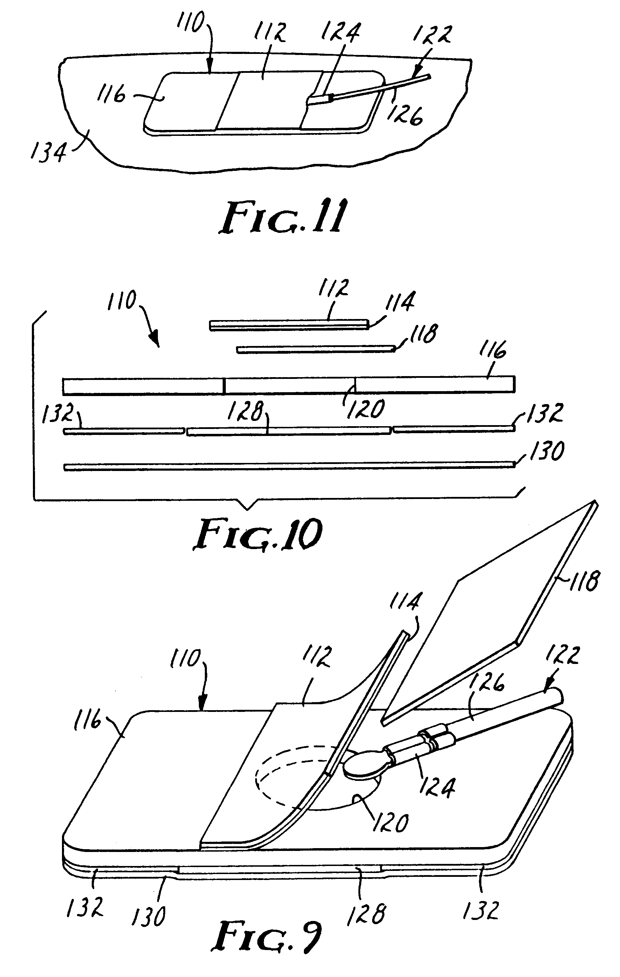

The biomedical electrode 10E illustrated in Figure 7 is similar to the biomedical electrode 10 illustrated in Figure 2. Biomedical electrode 10E omits electrically conductive 20 (shown in Figure 2) and since electrically conductive adhesive 20 has been omitted release liner 18 need only be release coated on the side facing adhesive 16. As in Figure 2, the biomedical electrode 10E of Figure 6 has a protective, electrically insulative web 12 which is secured to the remainder of the biomedical electrode 10E with a layer of adhesive 16. It is preferred that layer of adhesive 16 be a pressure sensitive adhesive. As stated above, release liner 18 covers a portion of the surface area of layer of adhesive 16 and is release coated at least on its top side facing layer of adhesive 16. An electrically conductive film 22 is positioned below both adhesive 16 and release liner 18. It is preferred that electrically conductive film be a metallic coated electrically conductive substrate such as an aluminum vapor coat of from 200 to 1200 angstroms thick on a 28% carbon loaded ethylene vinyl acetate substrate. The metallic vapor coat should be on the top side of the electrically conductive film 22 facing said release liner 18. Generally, the electrical resistance over the length of the electrically conductive film should be less than or equal to 1 ohms per inch (0.39 ohms per centimeter) of electrically conductive film 22. An electrically conductive adhesive 24 of the same as described with respect to Figure 2, is than located below electrically conductive film 22 opposite the release liner 18 or layer of adhesive 16. Optionally, a second release liner (not shown) may be then applied to electrically conductive adhesive 24. The electrode is utilized by after first removing the release liner from electrically conductive adhesive 24 if any, applying the biomedical electrode to the body with electrically conductive adhesive 24. Protective web 12 may then be lifted and release liner 18 removed. Lead wire 14 is then inserted into the located where release liner 18 was removed and protective web 12 is secured over lead wire 14. The conductor plate 30 part of the noninsulated portion 26 of lead wire 14 is held in place by adhesive 15 and makes electrical contact with electrically conductive film 22. Insulative portion 28 of lead wire 14 is contained under the edge of protective web 12 in order to have a completely insulated biomedical electrode 10E system.

The biomedical electrode 10F illustrated in Figure 8 is similar to the biomedical electrode 10D illustrated in Figure 6. Biomedical electrode 10F has a top layer consisting of a protective electrically insulative web 32 which in a preferred embodiment is an electrically insulative plastic sheet. The bottom side of protective web 32 is coated with an electrically conductive metallic coating 34 which in a preferred embodiment is aluminum which has been vapor coated onto the protective web 32 preferably in thicknesses of from 700 to 1200 angstroms. A release liner 18 with the release agent facing downward is positioned adjacent to the metallic coating 34 and covers a portion of the surface area of the biomedical electrode 10F. An electrically conductive adhesive 24 of the same type as described in Figure 2 is then located below release liner 18 and is directly attached to release liner 18 or metallic coating 34. In use, biomedical electrode 10F may be applied to the body by electrically conductive adhesive 24. Release liner 18 may be removed and an electrical lead wire (not shown) may be inserted in place of the release liner 18. Once the electrical lead wire is placed, the protective web 32 may be resecured over the top of the electrical lead wire. Electrically conductive adhesive 24 holds both the electrical lead wire and protective web 32 in place. Metallic coating 34 over the lower surface of protective 32 helps distribute the electrical current supplied from or to the electrical lead wire over the entire surface area of biomedical electrode 10F. In a preferred embodiment, protective web 32 with metallic coating 34 and release liner 18 extend beyond one edge of electrically conductive adhesive 24 so that they may be easily grasped to facilitate easy removal of release liner 18 when required.

Although biomedical electrode 10 has been illustrated as being in generally rectangular shaped it is to recognized and understood that any suitable shape, size or configuration of biomedical electrode 10 may be utilized to fit or suit the particular environment or body part to which it is adapted to be applied.

Figures 9 and 10 illustrate the basic construction of the biomedical electrode 110 of the present invention. A protective web 112 is applied by a pressure – sensitive adhesive 114 to a backing material 116. A removable release liner 118 is positioned between the protective web 112 and its associated pressure – sensitive adhesive 114 and backing material 116. Backing material 116 contains an aperture 120 which the protective web covers. An electrical lead wire 122 having a conductive portion 124 and an insulative portion 126 is insertable into the biomedical electrode with the conductive portion 124 of lead wire 122 positioning itself within aperture of backing material 116. Although not absolutely required, it is preferred that lead wire 122 have an insulative portion 126 such that when the conductive portion of lead wire 122 is positioned within aperture 120 and covered with protective web 112 only the insulated portion 126 of lead wire 122 is exposed. An electrically conductive adhesive 128 is located on the backing material 116 below aperture 120. The electrically conductive adhesive 128 positioned in this manner will contact the conductive portion 124 of lead wire 122 when the lead wire is inserted into aperture 120. A second removable liner 130 is positioned below electrically conductive adhesive 128 to be removed before the biomedical electrode 110 is applied to the body. It is only necessary that electrically conductive adhesive 128 be positioned under aperture 120 in backing material 116. Electrically conductive adhesive 128, of course, could cover the entire surface area of backing material 116. Electrically conductive adhesive 128, of course, could cover the entire surface area of backing material 116. If this were the case, no other adhesive in the biomedical electrode would be required. However, since electrically conductive adhesives may be more expensive than other adhesives. In a preferred embodiment, electrically conductive adhesive 128 covers only the general area of backing material 116 which is under aperture 120. Another pressure – sensitive adhesive 132 is then applied to the remainder of the surface area of backing material 116 to enable the biomedical electrode to be secured to the body when it is utilized. Alternatively, pressure – sensitive adhesive 132 could be applied to the entire surface area of backing material 116 and the portion under aperture 120 would be removed when aperture 120 was cut into backing material 116.

Although shown in generally rectangular shape, biomedical electrode 110 by appropriate sizing or trimming of backing material 116 may be any appropriate shape desired in order to conform or be located in any particular spot on the body. Aperture 120 is shown circular in nature and central within backing member 116 neither is absolutely required. Aperture 120 could, for example, be elongated to more appropriately conform to the conductive portion 124 of lead wire 122 and need to be centrally located within the backing material 116. As illustrated, protective web 112 covers generally only the area of backing material 116 over aperture 120. In other embodiments, protective web 112 could, of course, cover more of the surface area of backing material 116 for the entire surface area of backing material 116, if desired.

In a preferred embodiment, protective web 112 is electrically insulative to confine the electrical signals used in the biomedical electrode 110 to the lead wire 122 or to the body. In a preferred embodiment, protective web 112 is approximately 2 mils (0.051 millimeters) thick and is preferably constructed from polyester film. Pressure sensitive adhesive 114 is preferably an acrylate adhesive. Release liner 118, which is removable, facilitates the lifting of protective web 112 away from backing material 116 in order to lead wire 122 may be inserted and protective web 112 subsequently applied securing a conductive portion 124 of lead wire 122 and the biomedical electrode 110. In a preferred embodiment, release liner 118 is a Polyslick material as manufactured by James River Corporation, H.P. Smith Division, Bedford Park, Illinois.

Backing material 116 may be constructed from any generally flat, body conformable, flexible material. In a preferred embodiment, backing material 116 is a foam material, and in a more preferred embodiment is a polyethylene foam, for example, a 0.003 inches thick polyethylene foam. Lead wire 122 may be copper wire whose insulative portion 126 is insulated with any suitable insulation as, for example, rubber or plastic. The conductive portion 124 of lead wire 122 may be a flat crimped conductor plate. The conductor plate is flat which facilitates the biomedical electrode 110 being of low profile, flat and conformable to the body. In most preferred embodiments of the biomedical electrode 110, the conductive portion 124 of lead wire 122 should be silver or silver plated and preferably have a chloride treatment. Conductive adhesive 128 operates to secure the biomedical electrode 110 to the body and to provide through electrical conductivity from the body to the biomedical electrode. It is preferred that conductive adhesive 128 have better cohesion than adhesion in order to facilitate the ease with which the biomedical electrode 110 may be removed from the body. In a preferred embodiment, conductive adhesive 128 is a conductive adhesive as described in U.S. Patent No. 4,554,924, Engel, Conductive Adhesive and Biomedical Electrode. Removable release liner 130 may also be Polyslick liner. Pressure sensitive adhesive 132 is also an acrylate adhesive.

Figure 11 illustrates the biomedical electrode 110 having been applied to a body 134. Lead wire 122 is shown being secured in the biomedical electrode 110 by protective web 112, its associated pressure−sensitive adhesive 114 (not shown) and the electrically conductive adhesive 128 (not shown) to which it contacts. Backing material 116 is secured to the body with electrically conductive adhesive 128 (not shown) and pressure−sensitive adhesive 132 (not shown). When this particular use for the biomedical electrode has been completed, protective web 112 may be pulled back releasing lead wire 122 from the biomedical electrode 110 allowing the reuse of lead wire 122 and another biomedical electrode 110. Since there are no silver components to the biomedical electrode except for the silver plating or silver containment of conductive portion 124 lead wire 122 and since the lead wire 122 may be reused many times, an economical biomedical electrode 110 is provided. Biomedical electrode is flatter and more conformable to the body contours and body movement than prior art electrodes. The biomedical electrode 110 relies on adhesive contact with a flat electrical conductor as opposed to rubber connector strips or snaps. The biomedical electrode 110 has a very low profile which makes it suitable to be worn under tight clothing and to be comfortable when slept upon or when leaned against as, for example, when sitting in a chair. The biomedical electrode 110 may be trimmed to virtually any size or shape to allow flexibility and adaptability in placement and location upon the body.

## Claims

1. A biomedical electrode (10) adapted to be applied to a body and adapted to be electrically and mechanically connected to an electrical lead wire (14), comprising:

    a protective, electrically insulative web (12);

    a first layer of adhesive (20 or 24) which is electrically conductive and positioned adjacent one side of said protective, insulative web (12), said one side adapted to be oriented toward said body; and

    a removable release liner (18) between said protective, electrically insulative web (12) and said first layer of adhesive (20 or 24), said release liner (18) having a release agent facing said first layer of adhesive (20 or 24), said release liner (18) covering only a portion of the surface area of said one side of said protective, electrically insulative web (12).

2. A biomedical electrode (10) as in claim 1 which further comprises an insertable electrical lead wire (14) having a distal uninsulated portion (26) and a proximate insulated portion (28), said distal uninsulated portion (26) of said electrical lead wire (14) being insertable between said first layer of adhesive (20 or 24) and said protective, electrically insulative web (12) and at least part of said proximate insulated portion (28) of said electrical lead wire (14) extending under an edge of said protective, electrically insulative web (12) whereby said removable release liner (18) may be discarded, said insertable electrical lead wire (14) may be positioned, said protective, electrically insulative web (12) may be secured over said uninsulated portion (28) of said electrical lead wire (14) and secured in place by said first layer of adhesive (20 or 24).

3. A biomedical electrode as in claim 1 which further comprises:

    a second layer of adhesive (16) which is pressure sensitive positioned between said protective, electrically insulative web (12) and said release liner (18);

    an electrically conductive film (22) positioned adjacent said first layer of adhesive (20) opposite said release liner (18); and

    a third layer of adhesive (24) which is electrically conductive and positioned adjacent said electrically conductive film (22) opposite said first layer of conductive adhesive (20) and adapted to be applied to said body.

4. A biomedical electrode (10) as in claims 1 or 3 which further comprises an additional release liner placed adjacent said first layer of adhesive (20 or 24) opposite said protective, electrically insulative web (12).

5. A biomedical electrode (10) as in claim 3 which further comprises an insertable electrical lead wire (14) having a distal uninsulated portion (26) and a proximate insulated portion (28), said distal uninsulated portion (26), of said electrical lead wire (14) being insertable between said first layer of adhesive (20 or 24) and said second layer of adhesive (16) and at least part of said proximate insulated portion (28) of

EP 0 259 006 B1

said electrical lead wire (14) extending under an edge of said protective, electrically insulative web (12) whereby said removable release liner (18) may be discarded, said insertable electrical lead wire (14) may be positioned, said protective, electrically insulated web (12) may be secured over said uninsulated portion (28) of said electrical lead wire (14) and secured in place by said first layer of adhesive and said second layer of adhesive.

6. A biomedical electrode (10) as in claim 1 which further comprises:
   a second layer of adhesive (16) positioned between said protective, electrically insulative web (12) and said release liner (18);
   an electrically conductive film (22) positioned adjacent said second layer of adhesive (16) opposite said release liner (18); and
   an insertable electrical lead wire (14) having a distal uninsulated portion (26) and a proximate insulated portion (28), said distal uninsulated portion (26) of said electrical lead wire (14) being insertable between said first layer of adhesive (20) and said electrically conductive film (22) and at least part of said proximate insulated portion (28) of said electrical lead wire (14) extending under an edge of said protective, electrically insulative web (12).

7. A biomedical electrode (10) as in claims 3 or 6 wherein said electrically conductive film (22) has a metallic coating.

8. A biomedical electrode (10) as in claim 1 which further comprises an electrically conductive metallic coating applied to said protective, electrically insulative web (12) between said protective, electrically insulative web (12) and said first layer of adhesive (20 or 24).

9. A biomedical electrode (110) adapted to be applied to a body (134), comprising:
   a backing material (116) having an aperture (120), having a top side adapted to be oriented away from said body (134) and having a bottom side adapted to be oriented toward said body (134);
   a protective web (112) at least partially secured to said top side of said backing material (116) and covering said aperture (120);
   a first removable liner (118) positioned between said protective web (112) and said backing material (116), said first removable liner (118) covering at least a portion of the area of said backing material (116) covered by said protective web (112) and covering said aperture (120);
   a first layer of adhesive (128) which is electrically conductive and positioned adjacent said bottom side of said backing material (116) and adapted to be applied to said body (134);
   a second removable liner (130) positioned adjacent said first layer of adhesive (128) opposite from said backing material (116); and
   a lead wire (122) having one end (124) adapted to be positioned within said aperture (120) from said top side of said backing material (116) so as to be adhered to said first layer of adhesive (128);
   whereby said second removable liner (130) may be removed and said biomedical electrode (110) may be applied to said body (134) with said first layer of adhesive (128) and whereby said first removable liner (118) may be removed and said lead wire (122) may be positioned with said one end (124) within said aperture (120) and secured by said protective web (112).

10. A biomedical electrode (110) as in claim 9 which further comprises a second layer of adhesive (114) positioned between said protective web (112) and said top side of backing material (116).

11. A biomedical electrode (110) as in claims 9 or 10 wherein said backing material (116) comprises a foam material.

**Patentansprüche**

1. Biomedizinische Elektrode (10), die so ausgeführt ist, daß sie an einem Körper angebracht und elektrisch sowie mechanisch mit einem elektrischen Leitungsdraht (14) verbunden werden kann, umfassend:
   eine schützende, elektrisch isolierende Gewebeschicht (12);
   eine erste Kleberschicht (20 oder 24), die elektrisch leitend und neben einer Seite der schützenden, elektrisch isolierenden Gewebeschicht (12) angeordnet ist, wobei die eine Seite zum Körper weisend ausgeführt ist;

9

EP 0 259 006 B1

und
einen abziehbaren Trennstreifen (18) zwischen der schützenden, elektrisch isolierenden Gewebeschicht (12) und der ersten Kleberschicht (20 oder 24), wobei der Trennstreifen (18) mit einem zur ersten Kleberschicht (20 oder 24) weisenden Trennmittel versehen ist, und wobei der Trennstreifen (18) nur einen Teil der Oberfläche der einen Seite der schützenden, elektrisch isolierenden Gewebeschicht (12) abdeckt.

2. Biomedizinische Elektrode (10) gemäß Anspruch 1, die weiterhin einen einführbaren, elektrischen Leitungsdraht (14) mit einem distalen, nichtisolierten Abschnitt (26) und einem benachbarten, isolierten Abschnitt (28) umfaßt, wobei der distale, nichtisolierte Abschnitt (26) des elektrischen Leitungsdrahtes (14) zwischen der ersten Kleberschicht (20 oder 24) und der schützenden, elektrisch isolierenden Gewebeschicht (12) eingeführt werden kann, und wobei mindestens ein Teil des benachbarten isolierten Abschnitts (28) des elektrischen Leitungsdrahtes (14) unter einen Rand der schützenden, elektrisch leitenden Gewebeschicht (12) geführt ist, so daß der abziehbare Trennstreifen (18) entfernt, der einführbare elektrische Leitungsdraht (14) angeordnet, die schützende, elektrisch isolierende Gewebeschicht (12) über dem nichtisolierten Abschnitt (28) des elektrischen Leitungsdrahtes (14) befestigt und mittels der ersten Kleberschicht (20 oder 24) fixiert werden kann.

3. Biomedizinische Elektrode gemäß Anspruch 1, die weiterhin folgendes umfaßt:
eine selbsthaftende Kleberschicht (16), die zwischen der schützenden, elektrisch isolierenden Gewe‐beschicht (12) und dem Trennstreifen (18) angeordnet ist;
einen elektrisch leitenden Film (22), der neben der ersten Kleberschicht (20) dem Trennstreifen (18) gegenüberliegend angeordnet ist; und
eine elektrisch leitende und neben dem elektrisch leitenden Film (22) gegenüber der ersten leitenden Kleberschicht (20) angeordnete Kleberschicht (24), die sie ausgeführt ist, daß sie am Körper ange‐bracht werden kann.

4. Biomedizinische Elektrode (10) gemäß Anspruch 1 oder 2, die weiterhin einen zusätzlichen Trenn‐streifen umfaßt, der neben der ersten Kleberschicht (20 oder 24) gegenüber der schützenden, elektrisch leitenden Gewebeschicht (12) angebracht ist.

5. Biomedizinische Elektrode (10) gemäß Anspruch 3, die weiterhin einen einführbaren, elektrischen Leitungsdraht (14) mit einem distalen, nichtisolierten Abschnitt (26) und einem benachbarten, isolierten Abschnitt (28) enthält, wobei der distale, nichtisolierte Abschnitt (26) des elektrischen Leitungsdrahtes (14) zwischen der ersten Kleberschicht (20 oder 24) und der zweiten Kleberschicht (16) eingeführt werden kann, und wobei mindestens ein Teil des benachbarten isolierten Abschnitts (28) des elektri‐schen Leitungsdrahtes (14) unter einen Rand der schützenden, elektrisch leitenden Gewebeschicht (12) geführt ist, so daß der abziehbare Trennstreifen (18) entfernt, der einführbare elektrische Leitungsdraht (14) angeordnet, die schützende, elektrisch isolierende Gewebeschicht (12) über dem nichtisolierten Abschnitt (28) des elektrischen Leitungsdrahtes (14) befestigt und mittels der ersten und der zweiten Kleberschicht fixiert werden kann.

6. Biomedizinische Elektrode (10) gemäß Anspruch 1, die weiterhin folgendes umfaßt:
eine zweite zwischen der schützenden, elektrisch isolierenden Gewebeschicht (12) dem Trennstreifen (18) gegenüberliegend angeordnete Kleberschicht (16); und einen elektrisch leitenden Film (22), der neben der zweiten Kleberschicht (16) gegenüber dem Trennstreifen (18) angeordnet ist; und
einen einführbaren, elektrischen Leitungsdraht (14) mit einem distalen, nichtisolierten Abschnitt (26) und einem benachbarten, isolierten Abschnitt (28), wobei der distale, nichtisolierte Abschnitt (26) des elektrischen Leitungsdrahtes (14) zwischen der ersten Kleberschicht (20) und dem elektrisch leitenden Film (22) eingeführt werden kann, und wobei mindestens ein Teil des benachbarten isolierten Ab‐schnitts (28) des elektrischen Leitungsdrahtes (14) unter einen Rand der schützenden, elektrisch leitenden Gewebeschicht (12) geführt ist.

7. Biomedizinische Elektrode (10) gemäß Anspruch 3 oder 6, dadurch gekennzeichnet, daß der elektrisch leitende Film (22) mit einem metallischen Überzug versehen ist.

8. Biomedizinische Elektrode (10) gemäß Anspruch 1, die weiterhin einen elektrisch leitenden metalli‐schen Überzug umfaßt, der auf der schützenden, elektrisch isolierenden Gewebeschicht (12) zwischen

10

der schützenden, elektrisch isolierenden Gewebeschicht (12) und der ersten Kleberschicht (20 oder 24) aufgebracht ist.

9.  Biomedizinische Elektrode (110), die so ausgeführt ist, daß sie an einem Körper (134) angebracht werden kann, umfassend:
    ein Trägermaterial (116) mit einer Öffnung (120), dessen Oberseite so ausgeführt ist, daß sie vom Körper (134) wegweist, und dessen Unterseite so ausgeführt ist, daß sie zum Körper (134) gerichtet ist;
    eine schützende Gewebeschicht (112), die zumindest teilweise an der Oberseite des Trägermaterials (116) befestigt ist und die Öffnung (120) abdeckt;
    einen ersten abziehbaren Streifen (118), der zwischen der schützenden Gewebeschicht (112) und dem Trägermaterial (116) angeordnet ist, wobei der erste abziehbare Streifen (118) zumindest einen Teil der Fläche des von der schützenden Gewebeschicht (112) abgedeckten Trägermaterials (116) und die Öffnung (120) abdeckt;
    eine erste elektrisch leitende und neben der Unterseite des Trägermaterials (116) angeordnete Kleberschicht (128), die so ausgeführt ist, daß sie am Körper (134) angebracht werden kann;
    einen zweiten abziehbaren streifen (130), der neben der ersten Kleberschicht (128) gegenüber dem Trägermaterial (116) angeordnet ist; und
    einen Leitungsdraht (122) mit einem Ende (124), das so ausgeführt ist, daß es von der Oberseite des Trägermaterials (116) aus in der Öffnung (120) so angebracht werden kann, daß es durch die erste Kleberschicht (128) fixiert ist;
    worauf der zweite abziehbare Streifen (130) entfernt und die biomedizinische Elektrode (110) mittels der ersten Kleberschicht (128) am Körper (134) angebracht werden kann, und worauf der erste abziehbare streifen (118) entfernt und der Leitungsdraht (122) mit dem einen Ende (124) in der Öffnung (120) angeordnet und mittels der schützenden Gewebeschicht (112) befestigt werden kann.

10. Biomedizinische Elektrode (110) gemäß Anspruch 9, die des weiteren eine zweite Kleberschicht (114) umfaßt, die zwischen der schützenden Gewebeschicht (112) und der Oberseite des Trägermaterials (116) angeordnet ist.

11. Biomedizinische Elektrode (110) gemäß Anspruch 9 oder 10, bei der das Trägermaterial (116) einen Schaumstoff enthält.

**Revendications**

1.  Electrode biomédicale (10) destinée à être appliquée à un corps et prévue pour être électriquement et mécaniquement connectée à un câble conducteur électrique (14), comprenant :
    une feuille protectrice électriquement isolante (12) ;
    une première couche d'adhésif (20 et 24) qui est électriquement conductrice et adjacente à une face de ladite feuille protectrice isolante (12), cette face étant destinée à être orientée vers ledit corps ; et
    une pellicule anti−adhérence détachable (18) placée entre ladite feuille protectrice electriquement isolante (12) et ladite première couche d'adhésif (20 ou 24), ladite pellicule anti−adhérence (18) comportant un agent anti−adhérence en regard de ladite première couche d'adhésif (20 ou 24), ladite pellicule anti−adhérence (18) recouvrant seulement une partie de la surface de ladite face de ladite feuille protectrice électriquement isolante (12).

2.  Electrode biomédicale (10) suivant la revendication 1, qui comprend en outre un câble électrique insérable (14) ayant une partie d'extrémité libre non isolée (26) et une partie principale isolée (28), ladite partie d'extrémité non isolée (26) dudit câble électrique (14) pouvant être insérée entre ladite première couche d'adhésif (20 ou 24) et ladite feuille protectrice électriquement isolante (12), et au moins une portion de ladite partie principale isolée (28) du dit câble électrique (14) s'étendant sous un bord de ladite feuille protectrice électriquement isolante (12), de sorte qu'on peut enlever ladite pellicule anti−adhérence détachable (18), on peut positionner ledit câble électrique insérable (14) et on peut fixer ladite feuille protectrice électriquement isolante (12) sur la dite partie non isolée (28) dudit câble électrique (14) et la tenir en place au moyen de ladite première couche d'adhésif (20 ou 24).

3.  Electrode biomédicale suivant la revendication 1, qui comprend en outre :
    une couche d'adhésif (16) sensible à la pression, placée entre ladite feuille protectrice électriquement isolante (12) et ladite pellicule anti – adhérence (18) ;

un film électriquement conducteur (22) adjacent à ladite première couche d'adhésif (20) à l'opposé de ladite pellicule anti – adhérence (18) ; et

une couche d'adhésif (24) qui est électriquement conductrice et adjacente audit film électriquement conducteur (22) à l'opposé de ladite première couche d'adhésif conducteur (20), et destinée à être appliquée audit corps.

4. Electrode biomédicale (10) suivant les revendications 1 ou 3, qui comprend en outre une pellicule anti – adhérence supplémentaire, adjacente à la dite première couche d'adhésif (20 ou 24) à l'opposé de ladite feuille protectrice électriquement isolante (12).

5. Electrode biomédicale (10) suivant la revendication 3, qui comprend en outre un câble électrique insérable (14) ayant une partie d'extrémité libre non isolée (26) et une partie principale isolée (28), ladite partie d'extrémité non isolée (26) dudit câble électrique (14) pouvant être insérée entre la dite première couche d'adhésif (20 ou 24) et ladite deuxième couche d'adhésif (16), et au moins une portion de ladite partie principale isolée (28) dudit câble électrique (14) s'étendant sous un bord de ladite feuille protectrice électriquement isolante (12), de sorte qu'on peut enlever ladite pellicule anti – adhérence détachable (18), on peut mettre en place ledit câble électrique insérable (14) et on peut fixer ladite feuille protectrice électriquement isolante (12) sur ladite partie non isolée (28) dudit câble électrique (14) et la maintenir en place au moyen de ladite première couche d'adhésif et de ladite deuxième couche d'adhésif.

6. Electrode biomédicale (10) suivant la revendication 1, qui comprend en outre :

une deuxième couche d'adhésif (16) placée entre ladite feuille protectrice électriquement isolante (12) et ladite pellicule anti – adhérence (18) ;

un film électriquement conducteur (22) adjacent à ladite deuxième couche d'adhésif (16), à l'opposé de ladite pellicule anti – adhérence (18) ; et

un câble électrique insérable (14) ayant une partie d'extrémité non isolée (26) et une partie principale isolée (28), ladite partie d'extrémité non isolée (26) dudit câble électrique (14) étant insérable entre ladite première couche d'adhésif (20) et ledit film électriquement conducteur (22), et au moins une portion de ladite partie principale isolée (28) dudit câble électrique (14) s'étendant sous un bord de ladite feuille protectrice électriquement isolante (12).

7. Electrode biomédicale (10) suivant les revendications 3 ou 6, dans laquelle ledit film électriquement conducteur (22) comporte un revêtement métallique.

8. Electrode biomédicale (10) suivant la revendication 1, qui comprend en outre un revêtement métallique électriquement conducteur appliqué à ladite feuille protectrice électriquement isolante (12), entre la dite feuille protectrice électriquement isolante (12) et ladite première couche d'adhésif (20 ou 24).

9. Electrode biomédicale (110) destinée à être appliquée à un corps (134), comprenant :

un support (116) ayant une ouverture (120), ayant une face supérieure destinée à être orientée à l'opposé dudit corps (134) et ayant une face inférieure destinée à être orientée vers ledit corps (134) ;

une feuille protectrice (112) au moins partiellement fixée à ladite face supérieure dudit support (116) et recouvrant ladite ouverture (120) ;

une première pellicule anti – adhérence détachable (118) placée entre ladite feuille protectrice (112) et ledit support (116), ladite première pellicule anti – adhérence détachable (118) recouvrant au moins une partie de la surface dudit support (16) recouverte par ladite feuille protectrice (112) et recouvrant ladite ouverture (120) ;

une première couche d'adhésif (128) qui est électriquement conductrice et adjacente à ladite face inférieure dudit support (116) et qui peut être appliquée audit corps (134) ;

une deuxième pellicule anti – adhérence détachable (130) adjacente à ladite première couche d'adhésif (128) à l'opposé dudit support (116) ; et

un câble conducteur (122) ayant une extrémité (124) qui peut être placée dans ladite ouverture (120) à partir de ladite face supérieure dudit support (116), de façon à être collée à ldite première couche d'adhésif (128) ;

de sorte que ladite deuxième pellicule anti – adhérence détachable (130) peut être enlevée et ladite électrode biomédicale (110) peut être appliquée audit corps (134) au moyen de ladite première couche d'adhésif (128), et de sorte que ladite première pellicule anti – adhérence détachable (118) peut être enlevée et le dit câble (122) peut être placé avec ladite une extrémité (124) dans ladite ouverture (120) et fixé par la dite feuille protectrice (112).

10. Electrode biomédicale (110) suivant la revendication 9, qui comprend en outre une deuxième couche d'adhésif (114) placée entre ladite feuille protectrice (112) et ladite face supérieure du support (116).

11. Electrode biomédicale (110) suivant les revendications 9 ou 10, dans laquelle ledit support (116) est en une matière en mousse.

FIG.4

FIG.6

FIG.1

FIG.2

FIG.3

FIG.5

*Fig. 7*

*Fig. 8*

*FIG. 11*

*FIG. 10*

*FIG. 9*